# NEUE EUROPÄISCHE PATENTSCHRIFT

(11) **EP 0 203 945 B2**
(45) Veröffentlichungstag und Bekanntmachung des Hinweises auf die Entscheidung über den Einspruch: **24.06.1998**
(45) Hinweis auf die Patenterteilung: 02.08.1989
(21) Anmeldenummer: 85905779.6
(22) Anmeldetag: 15.11.1985
(51) Int. Cl.: A61M 29/02

(54) **DILATATIONSKATHETER**
DILATATION CATHETER
CATHETER DE DILATATION

(30) Priorität: 23.11.1984 DE 3442736
(43) Veröffentlichungstag der Anmeldung: 10.12.1986
(73) Patentinhaber: BONZEL, Tassilo, 36039 Fulda (DE)
(72) Erfinder: BONZEL, Tassilo, 36039 Fulda (DE)
(74) Vertreter: RACKETTE Partnerschaft Patentanwälte
(86) Internationale Anmeldenummer: DE8500479
(87) Internationale Veröffentlichungsnummer: WO8603129

(56) Entgegenhaltungen:
- DE-A- 3 010 743
- DE-A- 3 334 174
- DE-C- 867 144
- FR-A- 329 354
- GB-A- 1 566 308
- SU-A- 627 828
- US-A- 4 198 981
- American Journal of Cardiology, Vol. 49, April 1, 1982, Seiten 1216 bis 1222, J.B. Simpson et al: "A new catheter system for coronary angioplasty"
- Acta Radiology, Vol. 57, Nov. 1986, Seiten 411 bis 416, B. Nordenström "Balloon catheter for percutaneous insertion in the vascular system" (Nordenström 62)
- Radiology, Vol. 85, Juli bis Dezember 1965, Seiten 256 bis 259

## Beschreibung

Die Erfindung betrifft einen Koronar- Dilatationskatheter gemäß dem Oberbegriff des Anspruchs 1.

Ein derartiger Dilatationskatheter ist in The American Journal of Cardiology, Vol. 49, April 1, 1982, Seiten 1216 bis 1222, beschrieben und wird zur Aufweitung von Verengungen in Gefäßen und Körperhohlräumen, insbesondere von Koronararterien, verwendet. An der Spitze eines solchen Dilatationskatheters ist ein aufblasbarer Ballon angebracht, der über ein im Katheter befindliches Innenlumen gefüllt oder entleert werden kann.

Bei dem bekannten Dilatationskatheter ist ein Tubus vorgesehen, der am vorderen Ende in einen Ballon übergeht. Durch das Balloninnere und den Tubus erstreckt sich bei dem bekannten Dilatationskatheter ein Schlauch, der über das vordere Ende des Ballons vorsteht und mit dem vorderen Ende des Ballons abgedichtet verbunden ist. Durch das Innere des Schlauches ist ein Führungsdraht geführt, der während der Operation gegenüber dem Ballon verschoben werden kann, so daß der Dilatationskatheter entlang dem Führungsdraht vorgeschoben oder zurückgezogen werden kann. Beim Auswechseln eines mit Hilfe eines Führungskatheters gelegten Dilatationskatheters ist es erforderlich, daß der Führungsdraht um eine Länge aus dem Körper des Patienten herausragt, die größer ist als die Länge des Dilatationskatheters mit seinem Tubus. Aus diesem Grunde ist die Handhabung des bekannten Dilatationskatheters schwierig, zumal die Reibungskräfte zwischen dem Führungsdraht und dem den Ballon und den Tubus ganz durchsetzenden Schlauch groß sind.

In der DE 33 34 174 A1 wird ein lenkbarer Führungsdraht für einen Koronar-Dilatationskatheter beschrieben, dessen Schaft in Längsrichtung durch eine Trennwand in einen Hohlraum für das Dilatationsmittel und einen Hohlraum für der Führungsdraht aufgeteilt ist.

Ein Dilatationskatheter zur Einführung in die Speiseröhre ist aus der DE-A1-3 010 743 bekannt, wobei das Führungselement durch ein nur wenig biegsames Gastroskop gebildet ist, auf dem der mit dem Ballon verbundene Schlauchabschnitt mit Hilfe von Gummiringen lösbar so fixiert ist, daß außerhalb des Korpers des Patienten die genaue Lage des Ballons auf dem Gastroskop für den vorgesehenen Eingriff festgeiegt werden kann. Ein Verschieben des fixierten Ballons auf dem Gastroskop ist nach dem Einführen des Gastroskopes in die Speiseröhre nicht mehr möglich, da einerseits die Relbung des mit den Gummiringen festgeklemmten Schlauchabschnittes auf dem Führungselement absichtlich sehr groß ist und andererseits eine Zug- und Druckfestigkeit des flexiblen Tubus nicht notwendig und erwünscht ist. Der Tubus dient bei dem bekannten Katheter ausschließlich zum Transport des Inflationsmediums und insbesondere nicht zum Ausüben von Zug- oder Vorschubkräften auf den Ballon.

In der US-A-4 198 981 ist ein gynäkologisches Instrument geoffenbart, das im festen Abstand zwei Ballone aufweist. Die äußere Oberfläche eines inneren steifen Rohres ist mit einem Rohrabschnitt fest verbunden, auf dem einer der Ballone angeordnet ist.

Ausgehend von dem oben erörterten Stand der Technik liegt der Erfindung die Aufgabe zugrunde, einen Dilatationsketheter zum Aufweiten von Koronararterien zu schaffen, der entlang einem Führungselement leicht geführt und einfach gegen einen anderen Dilatationakatheter ausgewechselt werden kann.

Diese Aufgabe wird erfindungsgemäß durch die Merkmale des unabhängigen Anspruchs gelöst.

Dadurch, daß der mit der Oberfläche des Führungsdrahtes in Berührung kommende Schlauchabschnitt lediglich etwa die Länge des Ballons aufweist und der Tubus nicht mehr den Führungsdraht und den diesen umgebenden Führungsschlauch umgibt, erhöht sich die Handhabbarkeit des Dilatationskatheters. Die Steuerbarkeit ist wegen des Fehlens der Reibungskräfte in einem langen Führungsschlauch erheblich verbessert. Außerdem braucht der Führungsdraht wegen des verhältnismäßig kurzen Schlauchabschnitts nicht mehr etwa um die gleiche Länge aus dem Körper des Patienten herausragen, wie die Länge des Dilatationskatheters beträgt.

Zweckmäßige Ausgestaltungen und Weiterbil dungen der Erfindung sind in den Unteransprüchen gekennzeichnet.

Die Erfindung wird nachstehend anhand des in der Zeichnung dargestellten Ausfürungsbeispiel erläutert Es zeigen:
Fig. 1 den vorderen Teil des erfindungsgemäß en Dilatationskatheters mit dem in den Ballon einmündenden Tubus,
Fig 2 einen Querschnitt durch den Dilatationskatheter im Bereich des neben dem Führungsdraht verlaufenden Tubus und
Fig. 3 einen Querschnitt durch den Dilatationskatheter im Bereich eines Goldmarkers im Ballon

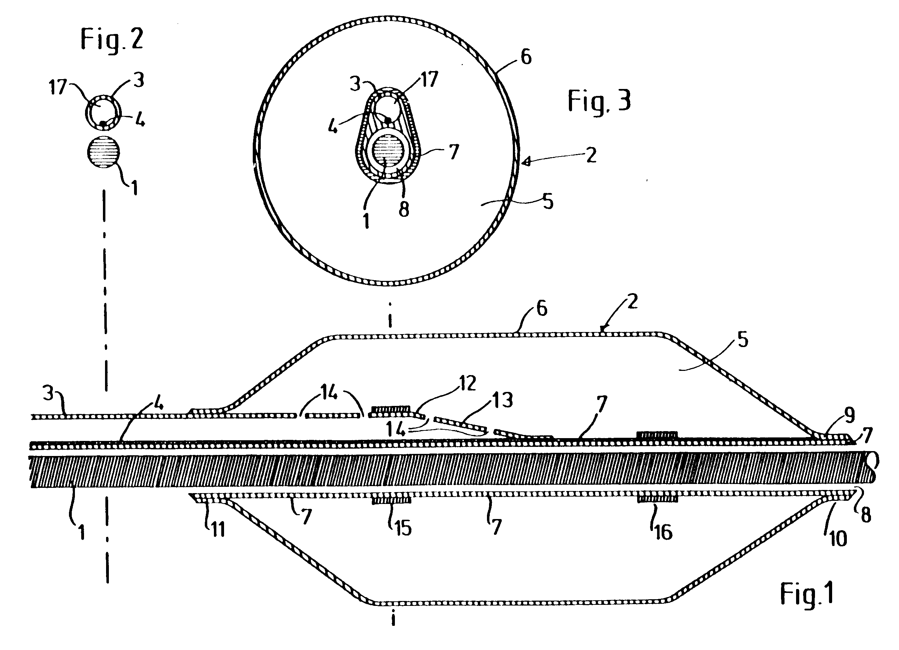

In Fig 1 ist der vordere Teil eines Dilatations katheters dargestellt, der mit Hilfe eines in der Zeichnung nicht dargestellten Führungskatheters mit einem Durchmesser von einigen Millimetern und einer Länge von etwa 1 m, beispielsweise von der rechten Leiste eines Patienten über die gesamte Länge der Arterie bis zur Aorta und zu den Koronararterien vorgeführt wird. Durch den in der Zeichnung nicht dargestellten Führungskatheter wird zunächst ein Führungsdraht 1 in die entsprechende Herzkranzarterie vorgeführt Ein Abschnitt des etwa 1 m langen Führungsdrahtes 1 ist in Fig sichtbar. Der Führungsdraht 1 dient als Instrumentierschiene zum Führen des Dilatationskatheters.

Der Dilatationskatheter verfügt über einen Ballon 2 und einen Tubus 3, der in Fig 1 abgeschnitten dargestellt ist und ebenfalls eine Länge in der Größenordnung von 1 m hat

In Fig 2 ist ein Schnitt durch den Führungsdraht 1 und den Tubus 3 dargestellt. Der Tubus 3 dient einerseits zur Übertragung von Schub- und Zugkräften zum Hin- und Herschieben sowie zum Drehen des Ballons 2 auf dem Führungsdraht 1. Aus diesem Grunde ist es zweckmäßig, wenn der Tubus 3 durch einen Stabilisierungsdraht 4 in der in den Figuren 1 bis 3 dargestellten Weise verstärkt ist. Neben seiner Aufgabe zur Übertragung von Kräften dient der Tubus 3 zum Einspritzen von Flüssigkeiten in das Balloninnere 5 zum Aufweiten des Ballons 2 und zum Absaugen von Flüssigkeiten, wenn der Ballondurchmesser verringert werden soll.

Wie in Fig. 1 zu erkennen ist, besteht der Ballon aus einer Ballonhülle 6 und einem Schlauchabschnitt 7, so daß der Ballon 2 einen Ballondurchgang 8 aufweist, der gegenüber dem Balloninneren 5 abgedichtet ist. Die Ballondurchführung 8 gestattet es, den Ballon 2 auf den Führungsdraht 1 aufzuschieben und dadurch auf dem Führungsdraht 1 zu führen.

In Fig. 3 erkennt man den im wesentlichen ringförmigen Querschnitt des Ballons 2 mit der Ballondurchführung 8, durch die sich der Führungsdraht 1 erstreckt. Um die auf den Tubus 3 ausgeübten Kräfte gut auf den Ballon 2 zu übertragen, reicht der Stabilisierungsdraht 4 bis in die Nähe des distalen Endes 9 des Ballons 2.

Wie man in Fig. 1 deutlich erkennt, ist am distalen Ende 9 des Ballons 2 die Ballonhülle 6 als Schlauchsegment 10 ausgebildet, das mit dem distalen Ende des Schlauchabschnittes 7 dicht verbunden ist. In ähnlicher Weise läuft die Ballonhülle 6 am proximalen Ende in ein Schlauchsegment 11 aus, das einerseits mit dem proximalen Ende des Schlauchabschnittes 7 abdichtend und andererseits mit dem Tubus 3 ahdichtend verbunden ist.

Das in Fig. 1 nach rechts weisende patientenseitige Ende 12 des Tubus 3 läuft in eine Verjüngung 13 aus, die am Schlauchabschnitt 7 befestigt ist. Sowohl in der Verjüngung 13 als auch im übrigen Bereich des patientenseitigen Endes 12 sind im Tubus 3 radiale Öffnungen 14 vorgesehen, über die die in den Tubus 3 eingespritzte Flüssigkeit vom Tubus 3 in das Balloninnere 5 des Ballons 2 gelangen kann.

In den Figuren 1 und 3 sind weiterhin Goldstreifen 15 und 16 dargestellt, die bei Röntgenaufnahmen zum Markieren der Lage des Dilatationskatheters dienen.

In Fig. 3 erkennt man einen Querschnitt durch den Ballon 2 im Bereich des Goldstreifens 15. Der Tubus 3 mit seinem Innenlumen 17 sowie der Schlauchabschnitt 7 mit dem Ballondurchgang 8 sind in dem in Fig. 3 gezeigten Bereich einstückig ausgebildet, so daß der Goldstreifen 15 im wesentlichen eine Ovalform und nicht die Form einer Ziffer 8 hat.

Der Führungsdraht 1 kann ein in der Zeichnung nicht dargestelltes zentrales Lumen für die Druckmessung oder ein Kontrastmittel enthalten Um den Reibungswiderstand zwischen dem Innern des Ballondurchgangs 8 und der Oberfläche des Führungsdrahtes 1 möglichst gering zu halten, kann die Innenseite des durch den Stabilisierungsdraht 4 versteiften Schlauchabschnitts 7 und/oder die Oberseite des Führungsdrahtes 1 mit einer Gleitschicht versehen sein.

Zur Dilatation von Herzkranzgefäßen wird durch den Führungskatheter zunächst der Führungsdraht 1 in die entsprechende Herzkranzarterie vorgeführt. Der Führungsdraht 1 liegt hierbei frei im Führungskatheter und ist somit gut dreh- und steuerbar. Zur anatomischen Orientierung sind zusätzlich ausreichende Kontrastmittelgaben möglich. Wenn der Führungsdraht 1 die Einengung in der Herzkranzarterie passiert hat, verbleibt die Spitze des Führungsdrahtes 1 weiterhin jenseits der Stenose im Herzkranzgefäß. Erst jetzt wird der erfindungsgemäße Dilatationskatheter außerhalb des Körpers auf den Führungsdraht 1 geschoben und an der durch den Führungsdraht 1 gebildeten Schiene durch den Führungskatheter in die Herzkranzarterie und über die Einengung vorgeschoben. Soll der Ballon 2 während der Operation gegen einen Ballon 2 mit größerer Ballonweite ausgewechselt werden, ist es auf einfache Weise möglich, den erfindungsgemäßen Dilatationskatheter zurückzuziehen, wobei der Führungsdraht 1 mit seinem vorderen Ende im Koronargefäß verbleibt und ein sicheres Vorführen des ausgewechselten Ballons erlaubt, ohne daß hohe Reibungskräfte zu überwinden sind und die Stenose erneut gesucht werden muß. Bei Verdacht auf mangelnde Stabilität des Dilatationserfolges kann der Führungsdraht 1 sogar für mehrere Stunden liegen bleiben, um zu einem späteren Zeitpunkt eine erneute Dilatation vorzunehmen. Das distale Ende 9 des Dilatationskatheters ist zur besseren Einführbarkeit in Gefäßeinengungen in der oben beschriebenen Weise flacher gestaltet.

Die Erfindung gestattet es, Ballons unterschiedlicher Länge, Weite und Wanddicke zur Aufnahme variabler Drücke vorzusehen und einfach auszuwechseln. Je nach den medizinischen Erfordernissen sind die Dilatationskatheter mit Tuben 3 unterschiedlicher Stärke und Biegsamkeit, die unterschiedliche Vorschübe gewährleisten, ausgerüstet. Für größere Dilatationskatheter ist ein in der Zeichnung nicht dargestelltes zusätzliches Innenlumen vorgesehen, dessen vorderes Ende bis zum distalen Ende 9 des Ballons 2 reicht und mit dem Gefäßinnenraum des operierten Patienten in Verbindung steht Hierdurch können Druckmessungen und Kontrastmittelinjektionen durchgeführt werden. Die Führungsdrähte 1 eines kompletten Instrumentariums weisen ebenfalls unterschiedliche Stärken und Biegsamkeiten auf. Die Führungsdrähte 1 verfügen über weiche flexible Spitzen, die kürzer oder länger sowie gerade oder gebogen sein können. Wenn im Ballon kein zusätzliches Innenlumen vorgesehen ist, kann in den Führungsdrähten ein bereits oben erwähntes zentrales Lumen für Druckmessungen und Kontrastmittelinjektionen vorgesehen sein.

## Patentansprüche

1. Koronar-Dilatationskatheter mit einem aufweitbaren Ballon (2), durch den sich ein auf einem Führung. selement (1) gehaltener Schlauchabschnitt (7) erstreckt, der mit dem distalen Ende (9) des Ballons (2) abgedichtet verbunden ist, und mit einem am proximalen Ende (11) des Ballons (2) abgedichtet ins Balloninnere (5) mündenden Tubus (3) zum Aufblähen des Ballons (2), wobei der Schlauchabschnitt (7) auf dem als flexiblen Führungsdraht (1) ausgebildeten Führungselement verschiebbar geführt ist
dadurch gekennzeichnet daß
die Längsachse des Tubus (3) gegenüber der Einführmündung am proximalen Ende des Schlauchabschnittes (7)
seitlich versetzt ist,
daß der Schlauchabschnitt (7) etwa die Länge des Ballons (2) aufweist, daß das proximale Ende (11) des Ballons (2) ebenfalls dichtend am Schlauchabschnitt (7) befestigt ist, und daß der Tubus (3) in Längsrichtung für die Übertragung von Schubund Zugkräften ausreichend steif ist, so daß durch ein Vorschieben oder ein Zurückziehen des Tubus (3) ein Verschieben des Schlauchabschnittes (7) mit dem daran befestigten Ballon (2) in beiden Richtungen auf dem Führungsdraht (1) ermöglicht ist.

2. Dilatationskatheter nach Anspruch 1 dadurch gekennzeichnet daß die Wandung des Tubus (3) an der Wandung des Schlauchabschnitts (7) angeformt ist.

3. Dilatationskatheter nach Anspruch 1 oder 2, dadurch gekennzeichnet, daß der Tubus (3) durch einen Stabilisierungsdraht (4) verstärkt ist.

4. Dilatationskatheter nach Anspruch 3, dadurch gekennzeichnet daß der Stabilisierungsdraht (4) sich im Innern des Ballons (2) entlang dem Mantel des Schlauchabschnittes (7) bis zum vorderen Ende (9) des Balloninnenraums (5) erstreckt.

5. Dilatationskatheter nach einem der vorstehenden Ansprüche, dadurch gekennzeichnet, daß die Ballonhülle (6) an ihren Befestigungsenden in Schlauchsegmente (10, 11) ausläuft, die parallel zum Schlauchabschnitt (7) auslaufen.

6. Dilatationskatheter nach einem der vorstehenden Ansprüche, dadurch gekennzeichnet, daß die Innenseite des Schlauchabschnittes (7) und /oder die Oberseite des Führungsdrahtes (1) mit einer Gleitschicht versehen ist.

## Claims

1. A coronary dilatation catheter comprising an expandable balloon (2) through which extends a length of tubing (7) which is held on a guide element (1) and which is scalingly connected to the distal end (9) of the balloon (2), and further comprising a tube (3) for inflating the balloon (2), the tube opening into the inside (5) of the balloon while being sealed at the proximal end (11) of the balloon (2), the tubing (7) being displaceably guided on the guide element which is formed by a flexible guide wire (1), **characterised in that** the longitudinal axis of the tube (3) is laterally displaced with respect to the inlet opening at the proximal end of the tubing (7), that the tubing (7) is only about as long as the balloon (2), that the proximal end (11) of the balloon (2) is likewise sealingly fixed to the tubing (7), and that the tube (3) is in the longitudinal direction sufficiently stiff to transmit pushing and pulling forces so that, by moving the tube (3) forwards or rearwards, the tubing (7), together with the balloon (2) attached thereto, may be displaced on the guide wire (1) in both directions.

2. A dilatation catheter according to claim 1, **characterised in that** the wall of the tube (3) is formed on the wall of the tubing (7).

3. A dilatation catheter according to claim 1 or 2, **characterised in that** the tube (3) is strengthened by a stabilising wire (4).

4. A dilatation catheter according to claim 3, **characterised in that** the stabilising wire (4) extends inside the balloon (2) along the wall of the tubing (7) up to the front end (9) of the balloon interior (5).

5. A dilatation catheter according to any of the preceding claims, **characterised in that** the balloon envelope (6) extends at its fixing ends into tube segments (10, 11) which extend parallel to the tubing (7).

6. A dilatation catheter according to any of the preceding claims, **characterised** in that the inside of the tubing (7) and/or the outside of the guide wire (1) are provided with an antifriction layer.

## Revendications

1. Cathéter de dilatation des artères coronaires comportant un ballonnet extensible (2), à travers lequel s'étend un tronçon de tuyau (7), qui est retenu sur un élément de guidage (1) et qui est relié d'une manière étanche à l'extrémité distale (9) du ballonnet (2), et comportant un tube (3) qui, au niveau de l'extrémité proximale (11) du ballonnet (2), débouche d'une manière étanche dans l'espace intérieur (5) du ballonnet, pour gonfler le ballonnet (2), le tronçon de tuyau (7) étant déplaçable d'une manière guidée sur l'élément de guidage réalisé sous la forme d'un fil de guidage flexible (1),
caractérisé en ce que
l'axe longitudinal du tube (3) est décalé latéralement par rapport à l'embouchure d'entrée au niveau de l'extrémité proximale du tronçon de tuyau (7),
que le tronçon de tuyau (7) possède approximativement la longueur du ballonnet (2), que l'extrémité proximale (11) du ballonnet (2) est également fixée d'une manière étanche au tronçon de tuyau (7) et que le tube (3) est suffisamment rigide dans la direction longitudinale pour la transmission de forces de poussée et de traction de sorte qu'un déplacement du tronçon de tuyau (7), sur lequel est fixé le ballonnet (2), dans les deux directions sur le fil de guidage (1) est possible au moyen d'une avance ou d'un retrait du tube (3).

2. Cathéter de dilatation selon la revendication 1, caractérisé en ce que la paroi du tube (3) est fixée par moulage sur la paroi du tronçon de tuyau (7).

3. Cathéter de dilatation selon la revendication 1 ou 2, caractérisé en ce que le tube (3) est renforcé par un fil de stabilisation (4).

4. Cathéter de dilatation selon la revendication 3, caractérisé en ce que le fil de stabilisation (4) s'étend à l'intérieur du ballonnet (2) le long de l'enveloppe du tronçon de tuyau (7) jusqu'à l'extrémité avant (9) de l'espace intérieur (5) du ballonnet.

5. Cathéter de dilatation selon l'une des revendications précédentes, caractérisé en ce que l'enveloppe (6) du ballonnet se termine, au niveau de ses extrémités de fixation, par des segments de tuyau (10, 11), qui se terminent en étant parallèles au tronçon de tuyau (7).

6. Cathéter de dilatation selon l'une des revendications précédentes, caractérisé en ce que la face intérieure du tronçon de tuyau (7) et/ou la face supérieure du fil de guidage (1) est pourvue d'une couche favorisant le glissement.
